# EUROPEAN PATENT APPLICATION

(11) **EP 2 351 735 A1**
(43) Date of publication of application: **03.08.2011**
(21) Application number: 09821954.6
(22) Date of filing: 14.10.2009
(51) Int. Cl.: C07C 317/04

(54) **SULFONE COMPOUND**

(30) Priority: 24.10.2008 JP 2008274932
(71) Applicant: Sumitomo Seika Chemicals Co. Ltd., Hyogo 675-0145 (JP)
(72) Inventor: TAKEUCHI Takeshi, Kako-gun Hyogo 675-0145 (JP); HIYAMA Takehiro, Kako-gun Hyogo 675-0145 (JP); LI Chun, Kako-gun Hyogo 675-0145 (JP); KANKI Toshihiko, Kako-gun Hyogo 675-0145 (JP)
(74) Representative: Albrecht, Thomas
(86) International application number: PCT/JP2009/067791
(87) International publication number: WO 2010/047257

(57) **Abstract**

It is an object of the present invention to provide an aprotic polar solvent mainly useful as a solvent for an electrochemical device, and having a comparatively low melting point and excellent thermal stability.

The present invention is a sulfone compound represented by the following formula (1), 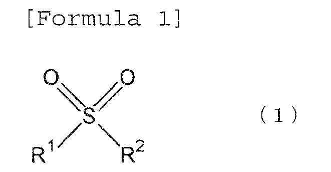 wherein, in the formula (1), R¹ and R² each represent a C3-C6 alkyl group, and R¹ and R² are different.

## Description

### TECHNICAL FIELD

The present invention relates to a sulfone compound. More particularly, the present invention relates to a sulfone compound mainly useful for a solvent for an electrochemical device and the like.

### BACKGROUND ART

Sulfone compounds are used as extraction solvents or various reaction solvents, and sulfone compounds with a high dielectric constant are also used as a solvent for an electrochemical device as an aprotic polar solvent. Specifically, proposed use of sulfone compounds as a solvent are seen in devices such as an electric double layer capacitor in which sulfone compounds such as sulfolane and sulfolane derivatives (e.g. 3-methyl sulfolane) are used as an electrolyte (Patent Document 1) ; and an electric double layer capacitor in which a mixed solvent of propylene carbonate and at least one of sulfolane and a sulfolane derivative such as 3-methyl sulfolane is used as an electrolyte (Patent Document 2).

Aprotic polar solvents used as a solvent for an electrochemical device and the like generally desirably have a low melting point, excellent thermal stability, and low viscosity. Depending on types of an electrochemical device, the existence of water within the system can become a problem. In such a case, a solvent with low solubility of water therein is preferably used.

However, sulfone compounds disclosed in Patent Documents 1 and 2 have a relatively high melting point, and therefore have problems such as a decrease in function in a low-temperature environment. Propylene carbonate used together with these sulfone compounds have problems such as inferior thermal stability and relatively high solubility of water in the propylene carbonate.

Patent Document 1: Japanese Kokai Publication Sho-62-237715(JP-A Sho-62-237715)
Patent Document 2: Japanese Kokai Publication Sho-63-12122(JP-A Sho-63-12122)

### DISCLOSURE OF THE INVENTION

### PROBLEMS TO BE SOLVED BY THE INVENTION

It is an object of the present invention to provide an aprotic polar solvent having a comparatively low melting point and excellent thermal stability.

The present invention relates to a sulfone compound represented by the following formula (1).

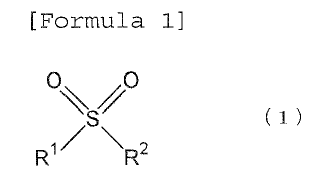

In the formula (1), R¹ and R² each represent a C3-C6 alkyl group, and R¹ and R² are different.

The C3-C6 alkyl group represented by R¹ is preferably a C3-C4 chain alkyl group, and particularly more preferably propyl group, isopropyl group, butyl group, or s-butyl group.

The C3-C6 alkyl group represented by R² is preferably a C4 branched-chain alkyl group, and particularly more preferably isobutyl group or s-butyl group.

Examples of the sulfone compound, represented by the formula (1), of the present invention include propyl isopropyl sulfone, propyl isobutyl sulfone, propyl s-butyl sulfone, propyl t-butyl sulfone, isopropyl butyl sulfone, isopropyl isobutyl sulfone, isopropyl s-butyl sulfone, isopropyl t-butyl sulfone, butyl isobutyl sulfone, butyl s-butyl sulfone, butyl t-butyl sulfone, isobutyl s-butyl sulfone, isobutyl t-butyl sulfone, s-butyl t-butyl sulfone, propyl isopentyl sulfone, isopropyl isopentyl sulfone, propyl isohexyl sulfone, isopropyl isohexyl sulfone, and the like.

Particularly, propyl isobutyl sulfone, propyl s-butyl sulfone, isopropyl butyl sulfone, isopropyl isobutyl sulfone, isopropyl s-butyl sulfone, butyl isobutyl sulfone, butyl s-butyl sulfone, and s-butyl isobutyl sulfone are preferably used.

The sulfone compound represented by formula (1) is prepared by, for example the following steps: a sulfide compound represented by formula (4) is prepared by reacting a thiol represented by formula (2) and an organic halide represented by formula (3), and the sulfide compound is oxidized using an oxidizing agent.

[Formula 2] R¹-SH (2)

In the formula (2), R¹ represents a C3-C6 alkyl group.

[Formula 3] R²-X (3)

In the above formula (3), R² represents a C3-C6 alkyl group, and is different from R¹ in the formula (2). X represents a halogen atom.

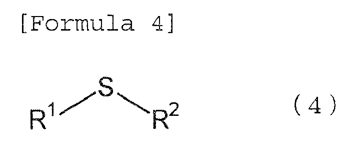

In the above formula (4), R¹ represents the same group as R¹ in the formula (2), and R² represents the same group as R² in the formula (3).

The thiol represented by the formula (2) and the organic halide may be ones commercially available.

Specific examples of the thiol include propyl thiol, isopropyl thiol, butyl thiol, and s-butyl thiol.

Specific examples of the organic halide include 2-bromobutane and 1-bromoisobutane.

In the reaction of the thiol represented by the formula (2) and the organic halide represented by the formula (3), the amount of the thiol used is preferably 0.5 to 10 moles, and more preferably 0.5 to 5 moles, with respect to 1 mole of the organic halide.

In the reaction of the thiol and the organic halide according to the present invention, a solvent may or may not be used. For example, a solvent may be used when the raw material is solid or the viscosity of the reaction liquid is too high to sufficiently stir. The solvent is not particularly limited, and examples thereof include alcohols such as methanol, ethanol, propanol, butanol, pentanol, hexanol, heptanol, octanol, nonanol, decanol, undecanol, dodecanol, tridecanol, tetradecanol, cyclohexanol, ethylene glycol, and propylene glycol; ethers such as diethyl ether, dipropyl ether, dibutyl ether, dipentyl ether, dihexyl ether, diheptyl ether, dioctyl ether, THF, tetrahydropyran, and 1, 4 -dioxane; nitriles such as acetonitrile, acrylonitrile, and propionitrile; ketones such as acetone, methyl ethyl ketone, methyl isobutyl ketone, and cyclohexanone; esters such as butyrolactone, caprolactone, hexanolactone, and ethyl acetate; sulfoxides such as dimethyl sulfoxide; hydrocarbons such as pentane, hexane, heptane, octane, nonane, decane, undecane, dodecane, cyclohexane, petroleum ether, benzine, kerosene, toluene, xylene, mesitylene, and benzene; and water. Among them, methanol and water are preferably used. Such solvents may be used alone or two or more kinds thereof may be used in combination.

The amount of the solvent used is not particularly limited, but is preferably 100 to 5000 parts by weight with respect to 100 parts by weight of the organic halide.

The reaction temperature is preferably 0°C to 200°C, and more preferably 10°C to 150°C. The reaction time is generally 1 to 30 hours.

In the production method of the sulfone compound of the present invention, specific examples of the oxidizing agent used for the oxidation of the sulfide compound represented by the formula (3) is not particularly limited, and examples thereof include potassium permanganate, chromic acid, oxygen, hydrogen peroxide water, organic peroxides such as 3-chloroperbenzoic acid, and the like. Particularly, hydrogen peroxide water is preferably used.

The amount of the oxidizing agent used is preferably 1.8 to 10 moles, and more preferably 2 to 5 moles, relative to 1 mole of the sulfide compound.

In the oxidation of the sulfide compound, a solvent may or may not be used. For example, a solvent may be used when the raw material is solid or the viscosity of reaction liquid is too high to sufficiently stir. The solvent is not particularly limited, and examples thereof include alkyl halides such as carbon tetrachloride, chloroform, dichloromethane, bromopropane, bromobutane, bromopentane, bromohexane, methyl iodide, ethyl iodide, and propyl iodide; ketones such as acetone, methyl ethyl ketone, methyl isobutyl ketone, and cyclohexanone; hydrocarbons such as pentane, hexane, heptane, octane, nonane, decane, undecane, dodecane, cyclohexane, petroleum ether, benzine, kerosene, toluene, xylene, mesitylene, and benzene; and water. Among them, alkyl halides and water are preferably used. Such solvents may be used alone or two or more kinds thereof may be used in combination.

The amount of the solvent used is not particularly limited, but is preferably 100 to 5000 parts by weight with respect to 100 parts by weight of the sulfide compound.

The reaction temperature is preferably 0°C to 200°C, and more preferably 10°C to 150°C. The reaction time is generally 1 to 30 hours.

The sulfone compound thus obtained is rinsed and separated, if needed, and can be isolated by distillation.

The sulfone compound of the present invention can be suitably used, for example, as a solvent for an electrochemical device, such as an electrolyte solvent.
Examples of the electrochemical device include lithium primary batteries, lithium secondary batteries, lithium ion batteries, fuel cells, solar cells, and electric double layer capacitors.

The sulfone compound of the present invention has a low solubility of water therein. Therefore, when the sulfone compound is used as the solvent for an electrochemical device, mixing with water is suppressed, which prevents occurrence of a decrease in current efficiency, an increase in internal pressure, and the like.
The sulfone compound of the present invention also has a comparatively low melting point and excellent thermal stability, and can be therefore safe to be used at a wide temperature range from low tempertures to high temperatures.
Further, the sulfone compound of the present invention has low viscosity, and therefore ion conductivity of an electrolyte can be significantly increased to achieve high electrical properties.

### EFFECTS OF THE INVENTION

The sulfone compound of the present invention is an aprotic polar solvent having a comparatively low melting point, excellent thermal stability, low viscosity, and a low solubility of water therein. Accordingly, the sulfone compound is useful mainly as a solvent for an electrochemical device.

### MODE(S) FOR CARRYING OUT THE INVENTION

The present invention is described below in more detail with reference to Examples. The present invention is not limited to the Examples.

### Example 1 [Synthesis of butyl isobutyl sulfone (BIBS)]

A 200-mL four-necked flask equipped with a stirrer, a thermometer, and a condenser was charged with 60 g (0.15 mol) of a 10% sodium hydroxide aqueous solution under a nitrogen atmosphere. An amount of 13.5 g (0.15 mol) of 1-butanethiol was gradually added to the solution while the solution was maintained at room temperature, and the solution was then stirred for one hour. Next, a 200-mL four-necked flask equipped with a stirrer, a thermometer, and a condenser was charged with 13.7 g (0.10 mol) of 1-bromo-2-methyl propane. All of the above sodium hydroxide aqueous solution of 1-butanethiol was added thereto, and the solution was then stirred at 60°C for two hours. The resulting solution was cooled to room temperature, and 50 mL of dichloromethane was added thereto and the resulting solution was stirred for 10 minutes. The dichloromethane layer was then isolated and rinsed once with 30 mL of ultrapure water. To the resulting dichloromethane layer was added 19.2 g (0.20 mol) of a 35% hydrogen peroxide solution. The solution was then stirred at 30°C for two hours, and the dichloromethane layer was distilled to give 16.0 g of butyl isobutyl sulfone in a colorless, transparent liquid form. The yield of the obtained butyl isobutyl sulfone was 90% with respect to 1-bromo-2-methyl propane.

The melting point and the exothermic onset temperature of the obtained butyl isobutyl sulfone were measured using a differential scanning calorimeter under a nitrogen atmosphere. In addition, viscosity thereof was measured using a rotational viscometer (product of Tokimec Inc., trade name "DISITAL VISCOMETER"). In addition, solubility of water in the butyl isobutyl sulfone was determined by measurement of a water content of the sulfone compound saturated with dissolved water using a Karl Fischer coulometric titrator.

The obtained butyl isobutyl sulfone was identified because it had the physical properties described below.

¹⁻H-NMR(400 MHz, solvent: CDCl₃) : 0.98 (t, J=7.4 Hz, 3H), 1.14 (d, J=6.8 Hz, 6H), 1.48 (m, 2H), 1.83 (m, 2H), 2.38 (m, 1H), 2.85 (d, J=6.5 Hz, 2H), and 2.95 (m, 2H)
Element analysis: C 53.9; H 10.2; S 18.0 (calculated value C 53.9; H 10.2; S 18.0)

### Example 2 [Synthesis of propyl isobutyl sulfone (PIBS)]

A 200-mL four-necked flask equipped with a stirrer, a thermometer, and a condenser was charged with 60 g (0.15 mol) of a 10% sodium hydroxide aqueous solution under a nitrogen atmosphere. An amount of 11.4 g (0.15 mol) of 1-propanethiol was gradually added to the solution while the solution was maintained at room temperature, and the solution was then stirred for one hour. Next, a 200-mL four-necked flask equipped with a stirrer, a thermometer, and a condenser was charged with 13.7 g (0.10 mol) of 1-bromo-2-methyl propane. All of the above sodium hydroxide aqueous solution of 1-propanethiol was added thereto, and the solution was then stirred at 60°C for two hours. The resulting solution was cooled to room temperature, and 50 ml of dichloromethane was added thereto and the resulting solution was stirred for 10 minutes. The dichloromethane layer was then isolated and rinsed once with 30 mL of ultrapure water. To the resulting dichloromethane layer was added 19.2 g (0.20 mol) of a 35% hydrogen peroxide solution. The solution was then stirred at 30°C for two hours, and the dichloromethane layer was distilled to give 14.5 g of propyl isobutyl sulfone in a colorless, transparent liquid form. The yield of the obtained propyl isobutyl sulfone was 88% with respect to 1-bromo-2-methyl propane.

The obtained propyl isobutyl sulfone was measured for the melting point, exothermic onset temperature, viscosity, and solubility of water therein by the same methods as those in Example 1. Table 1 shows the results.

The obtained propyl isobutyl sulfone was identified because it had the physical properties described below.

¹⁻H-NMR (400 MHz, solvent: CDCl₃) : 1.09 (t, J=7.4 Hz, 3H), 1.14 (d, J=6.7 Hz, 6H), 1.89 (m, 2H), 2.38 (m, 1H), 2.85 (d, J=6.6 Hz, 2H), 2.93 (m, 2H)
Element analysis: C 51.2; H 9.8; S 19.5 (calculated value C 51.2; H 9.8; S 19.5)

### Example 3 [Synthesis of isopropyl isobutyl sulfone (IPIBS)]

A 200-mL four-necked flask equipped with a stirrer, a thermometer, and a condenser was charged with 60 g (0.15 mol) of a 10% sodium hydroxide aqueous solution under a nitrogen atmosphere. An amount of 11.4 g (0.15 mol) of 2-propanethiol was gradually added to the solution while the solution was maintained at room temperature, and the solution was then stirred for one hour. Next, a 200-mL four-necked flask equipped with a stirrer, a thermometer, and a condenser was charged with 13.7 g (0.10 mol) of 1-bromo-2-methyl propane. All of the above sodium hydroxide aqueous solution of 2-propanethiol was added thereto, and the solution was then stirred at 60°C for two hours. The resulting solution was cooled to room temperature, and 50 ml of dichloromethane was added thereto and the resulting solution was stirred for 10 minutes. The dichloromethane layer was then isolated and rinsed once with 30 mL of ultrapure water. To the resulting dichloromethane layer was added 19.2 g (0.20 mol) of a 35% hydrogen peroxide solution. The solution was then stirred at 30°C for two hours, and the dichloromethane layer was distilled to give 11.2 g of isopropyl isobutyl sulfone in a colorless, transparent liquid form. The yield of the obtained isopropyl isobutyl sulfone was 85% with respect to 1-bromo-2-methyl propane.

The obtained isopropyl isobutyl sulfone was measured for the melting point, exothermic onset temperature, viscosity, and solubility of water therein by the same methods as those in Example 1. Table 1 shows the results.

The obtained isopropyl isobutyl sulfone was identified because it had the physical properties described below.

¹⁻H-NMR (400 MHz, solvent: CDCl₃) : 1.15 (d, J=6.7 Hz, 6H), 1.39 (d, J=6.8 Hz, 6H), 2.41 (m, 1H), 2.81 (d, J=6.5 Hz, 2H), 3.06 (m, 1H)
Element analysis: C 51.2; H 9.8; S 19.5 (calculated value C 51.2; H 9.8; S 19.5)

Table 1 shows the results of the measurement of the melting point, exothermic onset temperature, viscosity, and solubility of water in a compound, of the compounds of Examples 1 to 3 and of compounds for comparison, namely propylene carbonate as Comparative Example 1 and sulfolane as Comparative Example 2.

**[Table 1]**

| | Compound | Melting point (°C) | Exothermic onset temperature (°C) | Viscosity (cp) | Solubility of water (25°C)(g/100g) |
|---|---|---|---|---|---|
| Example 1 | BIBS | 1 | 200 | 8(25°C) | 3.7 |
| Example 2 | PIBS | 8 | 206 | 7(25°C) | 4.7 |
| Example 3 | IPIBS | -20 | 187 | 7(25°C) | 6.3 |
| Comparative Example 1 | Propylene carbonate | -49 | 73 | 3(25°C) | 8.4 |
| Comparative Example 2 | Sulfolane | 29 | 210 | 10(30°C) | Freely mixed |

### INDUSTRIAL APPLICABILITY

According to the present invention, an aprotic polar solvent that is useful mainly as a solvent for an electrochemical device, and has a comparatively low melting point and excellent thermal stability can be provided.

## Claims

1. A sulfone compound represented by the following formula (1), 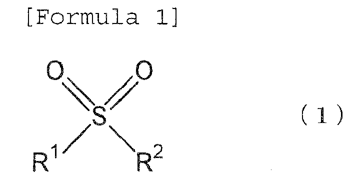 wherein, in the formula (1), R¹ and R² each represent a C3-C6 alkyl group, and R¹ and R² are different.

2. The sulfone compound according to claim 1, wherein, in the formula (1), R¹ is propyl group, isopropyl group, butyl group, or s-butyl group, and R² is isobutyl group or s-butyl group.
